# EUROPEAN PATENT APPLICATION

(11) **EP 2 564 804 A1**
(43) Date of publication of application: **06.03.2013**
(21) Application number: 11774213.0
(22) Date of filing: 29.04.2011
(51) Int. Cl.: A61C 3/14, A61B 17/28

(54) **ERGONOMIC TOOL FOR ATRAUMATIC TOOTH EXTRACTION**

(30) Priority: 29.04.2010 BR PI1001125
(71) Applicant: Vale, Adalberto De Carvalho, 37200-000 Lavras - MG (BR)
(72) Inventor: Vale, Adalberto De Carvalho, 37200-000 Lavras - MG (BR)
(74) Representative: Gonella, Mario
(86) International application number: PCT/BR2011/000140
(87) International publication number: WO 2011/134035

(57) **Abstract**

An ergonomic tool for atraumatic tooth extraction consists in an innovative tool that solves all problems related to tooth extraction which, by its own nature, is a traumatic and lengthy process that requires the prolonged application of strong physical forces and involves high risks of iatrogenic effects. An ingenious combination of two components - holding pliers that firmly keep the tooth to be extracted fixed between the active tips thereof before, during and after avulsion, and an extractor device which, by means of a lever and millimetric screw system, slowly applies strong forces for extracting the tooth without physical effort - makes the following contributions over the prior art: 1. The tool eliminates the numerous iatrogenic effects inherent to traditional tooth extraction, protecting the alveolar edges from injury, avoiding bleeding and any possibility of causing lockjaw in the patient; 2. It has near-zero risk of iatrogenic effects, such as crown breakage and the consequences thereof, as well as temporo-mandibular accidents; 3. It reduces the duration of tooth extraction procedures from about 40 to 4 minutes and the duration of cicatrisation to about 3 days; 4. It does not require physical effort of the dental surgeon; 5. It is the only tool that allows delicate interventions that are practically impossible using a forceps, making it possible to extract teeth with the cementoblast intact, for treatment outside the body, and to re-implant the teeth with absolute safety and very high rates of success; 6. It makes it possible to lift the teeth a few millimetres, with absolute safety, for orthodontic procedures of great value for health and aesthetic appearance; 7. Due to the small size of the tool, it makes tooth extraction possible even when the maximum mouth opening of the patient is reduced, as a result of lockjam, angular cheilitis or small size of the mouth, for example in children, requiring only 23 mm between the incisal edges of the maxillary central incisors and the incisal edges of the mandibular central incisors; 8. It has a the extraction of the third and fourth molars; 9. It does not require the use of electric or pneumatic drills for drilling crowns, shortening surgery time and reducing patient discomfort; 10. It reduces the cost of dental treatment, increasing the number of people that can benefit from dental care; 11. It has a low cost, since a single tool carries out all tooth extraction or prosthesis and pin withdrawal operations, unlike the present tooling, which requires a collection of more than ten tools; 12. It can be used in scientific veterinary research and medecine, for extracting teeth of small, medium and large size animals that are difficult to handle; 13. It is very easy to use, dispensing with long training periods; 14. Due to the system of universal adjustable pliers with active tips, it allows tooth extraction without support on any of the proximal teeth.

## Description

### Fields of this patent.

Tooth extraction.
Tooth extraction with minimal use of physical force.
• Elimination of alveolar and other iatrogenic lesions in tooth extraction.
• Extraction of dentures without use of blows.
• Ergonomics in tooth extraction implements.
• Productivity in dentistry by reducing the time for tooth extraction operations.
• Savings on dental implements.

### Prior art.

From its earliest beginnings, dentistry has had a tool which has practically become its symbol - the forceps for tooth extractions.

Forceps to the specialist, pincers to the layman.

In fact, from its earliest beginnings, when there was almost no dental hygiene and virtually no technique for extracting teeth, with abscesses or unbearable pain, it was the emergency solution; extraction of all the teeth for use of dentures was the planned solution.

Despite the great progress at present in dentistry, for various reasons tooth extraction or exodontia is still practiced: to eliminate unsalvageable teeth - or the remains of their roots, to eliminate unnecessary teeth such as the third molar or wisdom tooth, and more recently to gain space in the dental arch for correction of problems of occlusion and to obtain esthetic results, as in orthodontics.

### Criticism of the prior art.

The forceps is a dental tool that requires lengthy application of large and varied manual forces.

It is a pliers, that is, a set of two levers joined at any point between their two ends, the shorter parts of these levers being the ones applied to the object, to take advantage of the phenomenon of multiplication of forces and grasp the teeth firmly, and the longer parts, or ends, are the ones where force is applied by closing the hands.

The first of these forces is of constant pressure so that the tooth is held firmly in the tool, without escaping and causing accidents, but without breaking it; seemingly simple, the forceps demands strength and sensitivity from dental surgeons.

Other manual forces are applied to perform turns and inclinations on all sides - the technical term being luxations - for the purpose of breaking the fibers which bind the tooth to the periodontium.

Another type of force seeks to make the tooth move in the direction of its longer axis - avulsion - and to be extracted from the alveolar cavity, once the ligaments to the periodontium have been broken.

To avoid various problems, such as damage to the alveolus, copious bleeding, breakage of the tooth crowns, long post-operative time and others, such forces applied in extremely adverse ergonomic conditions demand great skill and physical strength from the dental surgeon.

In the specific case of breakage of a tooth crown, extraction of the roots becomes a complicated, painful and expensive operation.

Some tooth extractions can take more than 45 minutes.

The actual estimate made by the dental surgeon includes such accidents, which increases the costs of the dental treatments.

Besides the problems mentioned above, the tooling is costly and manifold: the basic tooling consists of a set of 8 forceps for adults and 2 forceps for children, besides many auxiliary tools. Thus, the prior art in exodontia, the forceps, is a source of risks, problems, costs, suffering of patients, and demands from surgeons the rare combination of great manual force and delicacy of movements, in more ergonomic conditions.

Due to such conditions, inconsistent with the great progress in dentistry in the past 20 years, various researchers have developed tools which employ pulleys, levers and screws, to promote tooth extraction with less physical force in a way that is safer, with more accuracy, less risk and fewer injuries.

Such devices are not manufactured in Brazil, they are very costly and the models available on the market are quite elementary, even though they are attractive metal pieces made of stainless steel with high quality.

Once a captive market was created, such devices, as often happens in the dental market, stayed in their initial forms without change, not encountering any competition.

### Advances brought to the prior art by the object of this patent.

The object of this patent, "ERGONOMIC TOOL FOR ATRAUMATIC TOOTH EXTRACTION", advances the prior art for various reasons that will be listed and demonstrated appropriately in the following.

We must first keep in mind that dentistry is a discipline of details, of fractions of a millimeter, a combination of art and technology, of health and beauty.

Few physical details of modern man are so exposed to social life as the teeth.

In an age when value is placed on the body, as at present, it is not only the healthiness, but also the beauty of a smile that decides a professional opportunity, a personal meeting, and even psychological health as the result of a positive self-image.

Modern dentistry is practiced in an age when awareness of dental hygiene is firmly established in younger persons; the major oral reconstructions are giving way to the practices of healthy occlusion and beauty.

Thus, the tooling, the techniques and the materials of dentistry must increasingly obey the grand precept of the health care sciences: "*primum non nocere*" - above all else, do no harm.

Tooth extraction can be extremely injurious; it can injure the alveoli, the temporo-mandibular joint, the internal hearing, the facial features, result in post-operative periods so long as to interfere with the economic and social life of the patients, and all of these drawbacks violate the basic principle of health care - *primum non nocere!*

The "**ERGONOMIC TOOL FOR ATRAUMATIC TOOTH EXTRACTION**" which is the object of this patent:
1. Eliminates the countless iatrogenic effects inherent to traditional tooth extraction, protects the alveolar edges from injury, does not cause bleeding, eliminates the possibility of causing lockjaw in the patient,
2. It has near-zero risk of iatrogenic effects, such as crown breakage and the consequences thereof, as well as temporo-mandibular accidents;
3. It reduces the duration of tooth extraction procedures from about 40 to 4 minutes and cicatrization to about 3 days;
4. It does not require physical effort of the dental surgeon;
5. It is the only tool that allows delicate interventions that are practically impossible using a forceps, making it possible for teeth to be extracted with the cementoblast intact and treated outside the body with absolute safety, and re-implanted with very high rates of success;
6. It makes it possible to lift the teeth a few millimeters, with absolute safety, for orthodontic procedures of great value for health and aesthetic appearance;
7. Due to the small size of the tool, it makes tooth extraction possible even when the maximum mouth opening of the patient is reduced, as a result of lockjaw, angular cheilitis or small size of the mouth, for example in children, requiring only 23 mm between the incisal edges of the maxillary central incisors and the incisal edges of the mandibular central incisors;
8. It has a small size, allowing the extraction of the third and fourth molars;
9. It does not require the use of electric or pneumatic drills for drilling crowns, shortening surgery time and reducing patient discomfort;
10. It reduces the cost of dental treatments, increasing the number of people that can benefit from dental care;
11. It has a low cost, since a single tool carries out all tooth extraction or prosthesis and pin withdrawal operations, unlike the present tooling, which requires a collection of more than ten tools;
12. It can be used in scientific research and veterinary medicine, for extracting teeth of small, medium and large size animals that are difficult to handle;
13. It is very easy to use, dispensing with long training periods;
14. Due to the system of universal adjustable pliers with active tips, it allows tooth extraction without support on any of the proximal teeth.

***Illustration and functioning*** of the "ERGONOMIC TOOL FOR ATRAUMATIC TOOTH EXTRACTION", the object of this patent.

The "ERGONOMIC TOOL FOR ATRAUMATIC TOOTH EXTRACTION" (1), *the object of this patent,* consists in an innovative and ingenious combination of two independent parts that can be coupled solely by juxtapositioning, of reduced dimensions, based on applications of the principles of basic machines - levers, screws and lines, without the need for pulleys, for a simple and effective operation, such that the multiplication of forces is done a millimeter at a time under total control and with no physical effort on the part of the dental surgeon, to avoid iatrogenic injuries and allow for delicate treatment operations and avulsion followed by re-implantation or not.

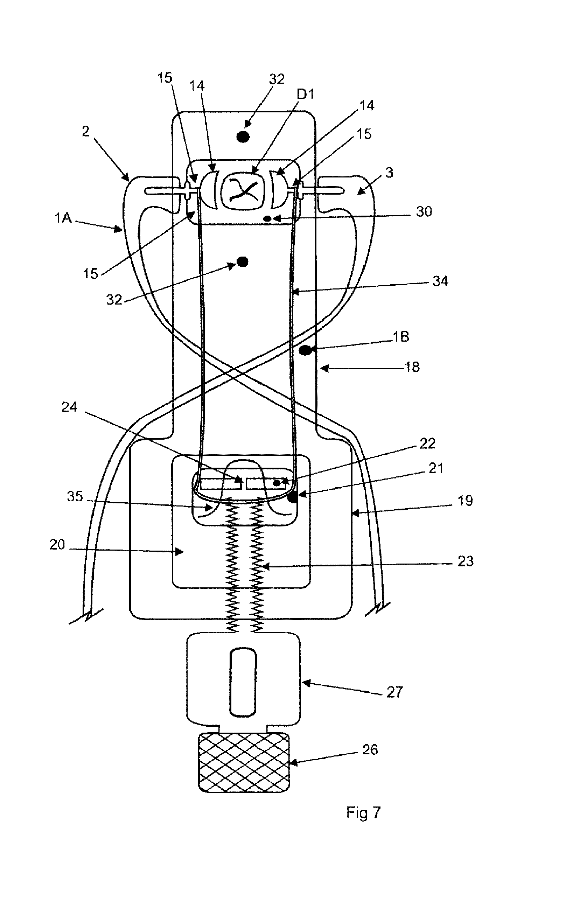

This set of two parts that are independent and can be coupled together has a group of devices, easily adjustable and removable from them, which shall be appropriately described and illustrated in the course of this specification; this group of devices consists of torque keys, active tips, support bases, a line fixing clip and a line, which will be illustrated in Fig. 7, but which will be cited as a line (34) beforehand in this specification.

To facilitate the understanding of the functioning of the "ERGONOMIC TOOL FOR ATRAUMATIC TOOTH EXTRACTION" (1), the functioning of each of its two constituent parts will first be described, after which details of same, and finally the functioning of the two parts coupled together will be described.

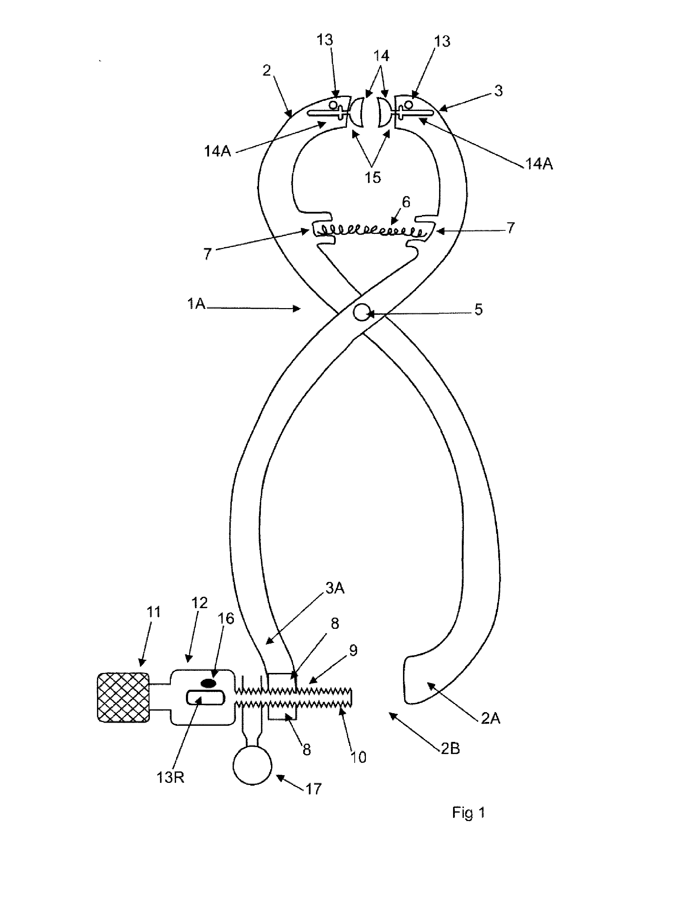

Figure 1 is a schematic top view, in almost true dimensions, but without keeping the proper proportions to amplify the details, for a better understanding, of the first of these parts making up the "ERGONOMIC TOOL FOR ATRAUMATIC TOOTH EXTRACTION", and known as the holding pliers (1A), in which we see the left shaft (2), the right shaft (3), the active tips (14), movable yet held fixed in position, so as not to become displaced during use, by the springs (13) that apply pressure to the active tips (14) when inserted in the openings (14A) of the two shafts; by the necks (15) of the active tips (14), that will be secured to the tips of the line (34), to be described further below.

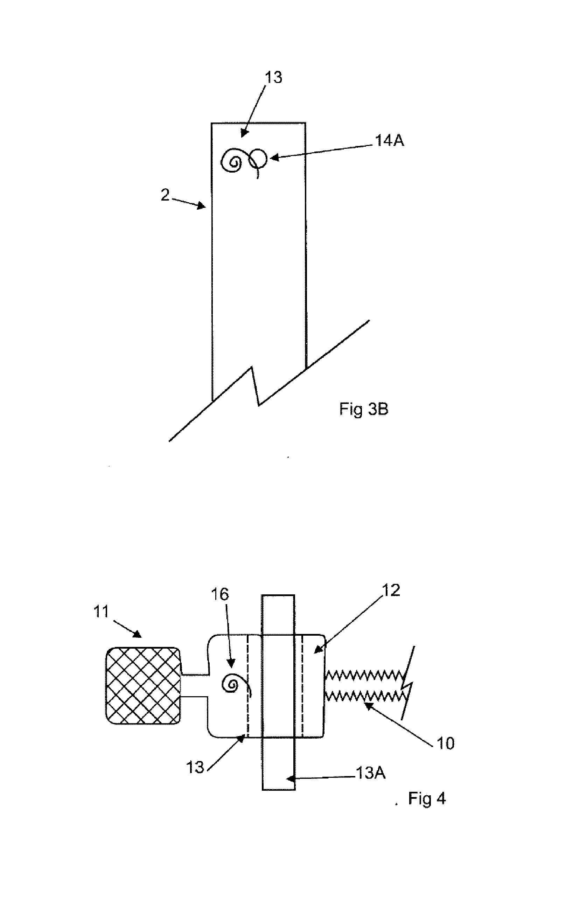

Also in Fig. 1 we see the spring which facilitates the operation (6), which is inserted in the seats (7), whose function is to keep the left shaft (2) and the right shaft (3) always opened, so that the holding pliers (1 A) is always ready for easy access to the area where it will be applied; we also see the distal end (3A) of the right shaft (3), the millimeter screw opening (9), the millimeter nut (8) through which passes, when turned, the millimeter screw (10), joined to the torque device (12), with its slot (13R), for passage of the torque key (13A), shown in Fig. 4; the spring (16) and the fast adjustment end (11), joined to the torque device (12).

In Fig. 1 we see that, when the fast adjustment end (11) is turned by the fingers of the operator, the torque device (12) and the millimeter screw (10) are also turned and the end of the millimeter screw (10) approaches, quickly and without needing effort, the lower end (2A) of the left shaft (2), causing the distance (2B) to diminish until it touches the distal end (2A) of the left shaft (2), these functions being performed delicately, with a simple turning of the tips of the index finger and thumb of the dental surgeon, who holds the two shafts in his hands, positioning the parts thereof, which contain the active tips (14), in the exact positions and locations of the teeth being treated; when certain that the active tips (14) are in their correct places, the torque key (13A) shown in Fig. 4 can pass through the slot (13R) of the torque device (12), so that the latter can be turned until the correct force is applied to the teeth via the active tips (14), which force is a consequence of the effect of the set of two levers, of intermediate support, formed by the left shaft (2) and the right shaft (3), which turn about the support tip realized by the axis of turning (5).

The torque key (13A), not shown in this Fig. 1, can pass through the slot (13) of the torque device (12), inside which it is held under pressure by the locking spring (16), so that as the torque device (12) is turned it can easily change position, forward or backward, so that its turning ability is not impeded by the patient's face or chin.

Also in Fig. 1 we see that, solely by the forces applied by the operator at the tips of his thumb and index finger to the torque key (13A), the millimeter screw (10) is turned and begins to apply force to the distal end (2A) of the left shaft (2), and this force can be regulated by the experience of the operator or controlled by torque meter (17), placed at the lower end (3A) of the right shaft (3).

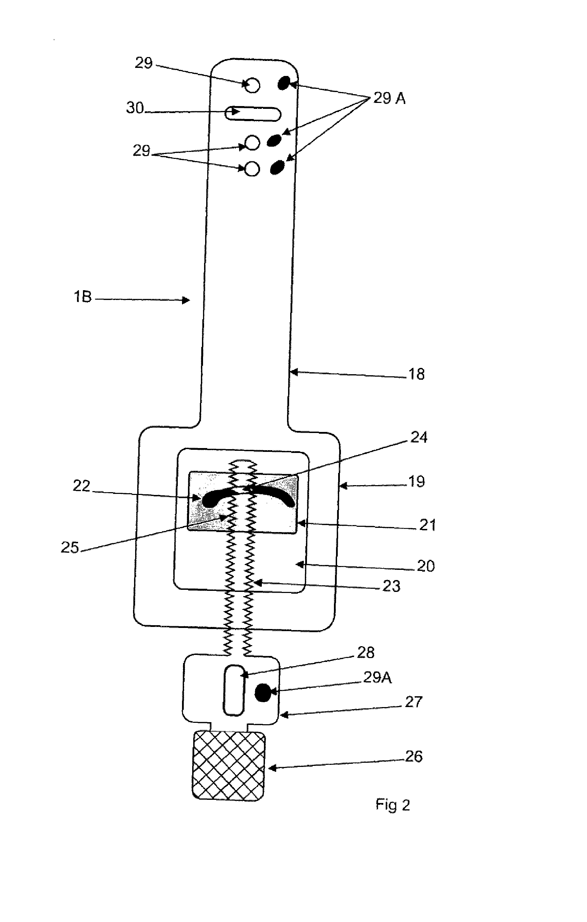

Figure 2 is a schematic top view, in near-true dimensions, but without keeping the proper proportions to amplify the details, for a better understanding, of the second part of the set of two parts making up the "ERGONOMIC TOOL FOR ATRAUMATIC TOOTH EXTRACTION" and known as the extractor (1 B); in this Fig. 2, we see the shaft (18), which widens in its terminal portion, which is outside the patient's mouth and forms the operating handle (19), hollowed out by the window (20), inside which travels the tensioning carriage (21) which, at its upper portion, has the fixator shoulder (22) for the line (34), illustrated in Fig. 7; said fixator shoulder (22) has, in its central portion, the groove (24); we also see the millimeter endless screw (23), which passes through the threaded opening (25) of the tensioning carriage (21), causing without physical effort on the part of the dental surgeon its movement toward the proximal part of the device or its retraction, the torque device (27), the slot (28) for passage and fixation of the torque key (13A), shown in Fig. 4, of the torque device (27), and the fast end (26).

In Fig. 2 we also see the slot (30) for movement of the line (34) illustrated in Fig. 7, the three threaded openings (29) where the screws for locking the support bases will pass, to be described in due course, and the three springs (29A) that will hold these screws in position; the function of the springs (29A) is to apply pressure to the screws inside the threaded holes (29) to hold them in place during the operation of the "ERGONOMIC TOOL FOR ATRAUMATIC TOOTH EXTRACTION".

The functioning of the regulating elements of the extractor (1 B) is as follows: the heights at which the latter will be braced against the teeth or gums bordering on the tooth being treated will be determined by two support bases which will be correctly positioned by screws held in place by the springs (29A) in regard to the height and other horizontal adjustments of the surgical field; the tensioning carriage (21) moves in the direction toward the distal part of the extractor (1 B), in the beginning by the rotation of the fast end (26), which is done gently and without the need to exert force, by the operator with the tips of his thumb and index finger, such that the tensioning carriage (21), through which passes the millimeter endless screw (23) that crosses the threaded opening (25) of the tensioning carriage (21), upon turning, causes the latter to move.

The functioning of the "ERGONOMIC TOOL FOR ATRAUMATIC TOOTH EXTRACTION" that comprises the holding pliers (1 A) and the extractor (1 B), properly coupled together by mere juxtapositioning, shall be explained after the detailed description of the parts making up the group of devices consisting of torque keys, active tips, support bases, line and line fixator clip, which will now be done.

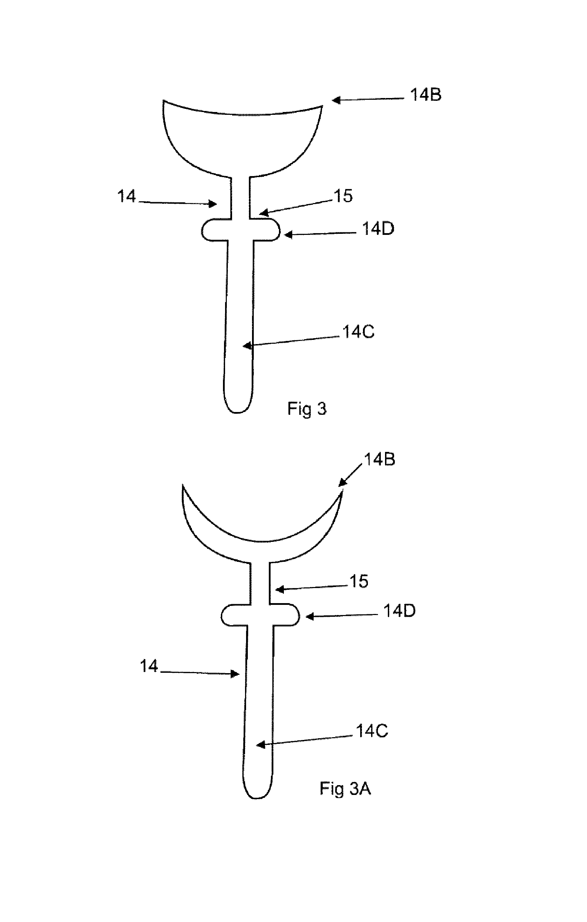

Figures 3 and 3A illustrate the active tips (14); Fig. 3 is a schematic front view and Fig. 3A is a schematic lateral view, with a 90 degree turn of the active tip (14) in relation to same, as shown in Fig. 3; in both, we see the grooves (14B), the necks (15) and the locating pin (14C), which engage in the openings (14A) of both the left shaft (2) and the right shaft (3), where they are firmly positioned by the action of the springs (13), as shown in Fig. 1, although they can be rotated by nearly 360°, so that the active tips (14) can be applied both to extractions of broad teeth (molars) and long teeth (incisors); we also see the shoulders (14D) of the locating pins (14C) of the active tips (14), whose functions are to hold the line (34), to be described in due course, in correct position between the necks (15) and the shoulders (14D).

Figure 3B is a schematic side view of part of the proximal end of the left shaft (2) per Fig. 1, where one sees an opening (14A) and a type of spring (13), in this case a classical model of a spiral spring that partially penetrates into an opening in order to apply pressure and hold onto any pin that is engaged in it; this way of keeping pressure on a pin inside an opening is only one example of how this effect can be accomplished, inasmuch as different means can be used without altering the scope of this patent, since what needs to be pointed out is the tremendous practical value of the firm fixation of the active tips (14) of Fig. 1; once the position is fixed by the operator, they remain so fixed and it is not necessary to make any other adjustment.

The active tips (14) preferably have the shape shown here, since this shape lends itself to all the needs for use of the "ERGONOMIC TOOL FOR ATRAUMATIC TOOTH EXTRACTION" and its single version is extremely economical; even so, its sizes and shapes can have other embodiments.

Figure 4 is a schematic top view which shows the torque key (13A), which can operate in the slot (13) illustrated in Fig. 1 or in the movement slot (30) of the line (34), to be demonstrated in due course and not yet illustrated in these figures, and the function of said torque key (13A) is to serve as a lever to impose torque, respectively, on the torque device (12) illustrated in Fig. 1 and on the torque device (27) illustrated in Fig. 2; inside these two devices there are, respectively, the spring (16) and the spring (29A), whose functions are to keep these parts fixed in the positions in which the operators place them, although by a simple intentional torque of the fingers these springs permit the movement of the keys, inside the slot (13) illustrated in Fig. 1 or in the slot for movement (30) of the line (34) illustrated in Fig. 2, and they will be turned between the index finger and thumb to exert the necessary torque on the parts of the "ERGONOMIC TOOL FOR ATRAUMATIC TOOTH EXTRACTION" (1) where this is implemented.

We see in Fig. 4 the millimeter screw (10), joined to the torque device (12) with its slot (13) for the passage of the torque key (13A) and the fast adjustment end (11) joined to the torque device (12) and the spring (16); in this figure it is seen that the torque key (13A) can assume any position inside the slot (13) and even be retracted.

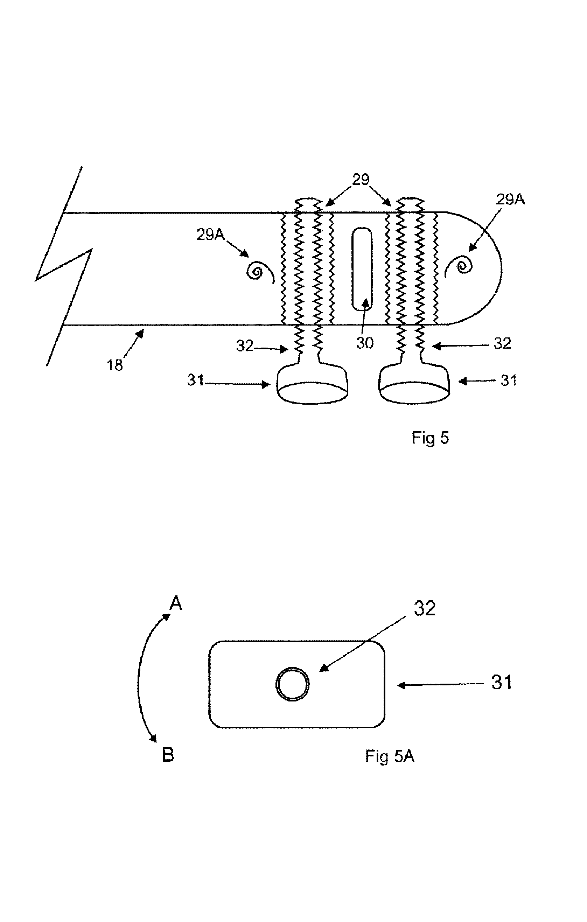

Figure 5 is a schematic side view of the proximal end of the shaft (18) of the extractor (1 B) of the "ERGONOMIC TOOL FOR ATRAUMATIC TOOTH EXTRACTION" (1) shown in top view in Fig. 2, which is the form in which the shaft (18) is introduced into the patient's oral cavity, to show the support bases (31) according to the same Fig. 2; in this Fig. 5, we see the springs (29A) that serve to hold the screws (32) firmly in the positions determined by the operator, both as regards the heights and the horizontal positions of same; we also see the movement slot (30), through which the line (34) will pass, coming from the tensioning carriage (21) as shown in Fig. 2, to wrap around the active tips (14) of the holding pliers (1A), shown in Fig. 1, by which the tooth (D1) will be extracted; we also see the screws (32) and the threaded openings (29) by which the screws (32) pass through the shaft (18), the support bases (31) with their flexible pads (33), which soften the contact with the teeth bordering on the tooth (D1) to be extracted or, if one or both of these are lacking, to soften the contact with the patient's gum, and serving as support for the "ERGONOMIC TOOL FOR ATRAUMATIC TOOTH EXTRACTION" (1) when it is being used.

Figure 5A is a top view of a support base (31), in which we see the screw (32) and the curved arrow "A-B" to show that the latter can be firmly positioned in any horizontal position, when rotated, to better adapt to the surgical field where it is applied.

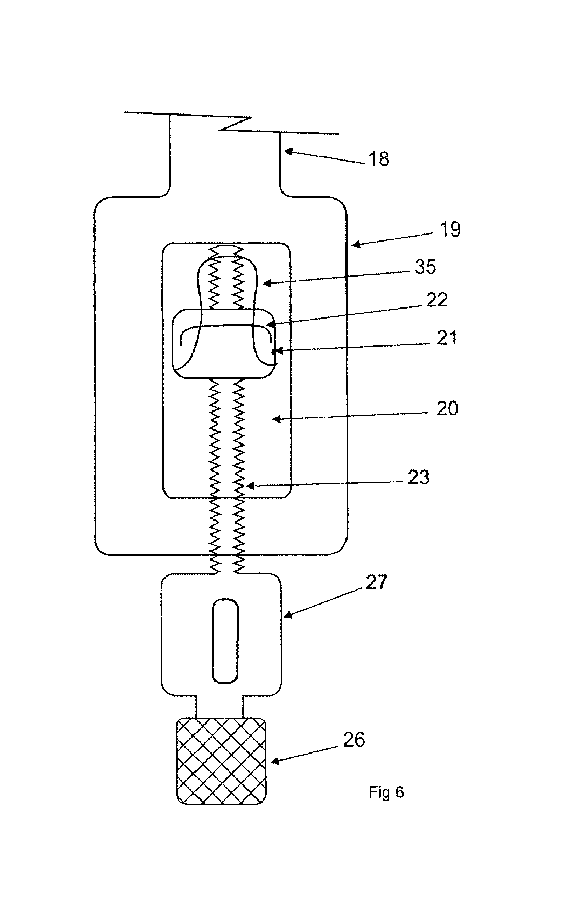

Figure 6 is a schematic top view, not heeding proportions, of the distal end of the extractor (1 B), which serves to show the tensioning carriage (21), already shown in Fig. 2, and the holding clip (35) for the line (34), which keeps the line (34) positioned without causing difficulties for the preliminary adjustment step of the "ERGONOMIC TOOL FOR ATRAUMATIC TOOTH EXTRACTION" (1), for the total or partial avulsion of any tooth or root; we also see a part of the shaft (18) and its operating handle (19), with its window (20), the fixator shoulder (22) for the line (34), not shown in this Fig. 6, the millimeter screw (23), which actuates the tensioning carriage (21), the torque device (27) and the fast end (26).

The holding clip (35) for the line (34) is one of the simple, yet very valuable details, typical of the care with which the devices of the implements used in dentistry need to be constructed; the holding clip (35) for the line (34) keeps the latter on the fixator shoulder (22) of the line (34), guaranteeing that the line (34) will not shift when the "ERGONOMIC TOOL FOR ATRAUMATIC TOOTH EXTRACTION" (1) is being adjusted, such that the line (34) will firmly enclose by lashing or any other suitable means the active tips (14), by their necks (15), and the teeth or crowns so extracted will not produce the risk of being swallowed by the patients.

Figure 7 is a schematic top view of the holding pliers (1A) and the extractor (1 B) placed on top of it, by mere superpositioning, in the operating position of the "ERGONOMIC TOOL FOR ATRAUMATIC TOOTH EXTRACTION" (1); the relationships between the active tips (14), the line (34), the tensioning carriage (21) and the movement slot (30) of the line (34) also will be shown in this Fig. 7. In this Fig. 7, we see the holding pliers (1A), the left shaft (2), the right shaft (3) and, through the movement slot (30) of the line (34), shown exaggerated in the figure to facilitate understanding of the functioning of the "ERGONOMIC TOOL FOR ATRAUMATIC TOOTH EXTRACTION" (1), thus allowing a view of the active tips (14) positioned at the ends of both the shafts and embracing the tooth to be extracted (D1), its necks (15), at which the two tips of the line (34) are lashed, whose size is calculated so that it is wrapped around and fixed in the shoulder (22) of the tensioning carriage (21); it is inferred from Fig. 7 that, except for the fact of having its two tips lashed to the necks (15) of the active tips (14), the line (34) is a device that has its loop free to move, which allows it to be positioned in the following way: once the holding pliers (1A) is positioned and firmly adjusted to the tooth to be extracted (D1), the extractor (1B) is placed on top of the holding pliers (1A), the line (34) is passed from the bottom upwardly through the movement slot (30) and also passed through the shoulder (22) of the tensioning carriage (21), which was previously positioned by the millimeter screw (23) in the extreme proximal position of the window (20) and held in this position by the holding clip (35); gently and without the need for physical effort, the dental surgeon begins to turn, with his thumb and index finger, the fast end (26) until the line (34) becomes slightly taut by the retraction of the tensioning carriage (21) toward the distal part of the window (20), at which time the surgeon begins the avulsion proper, by actuating, likewise with his thumb and index finger, the torque key (13A) and operates the torque device (27) and the line (34), by virtue of the force that the system of levers created by this torque device (27), acting on the millimeter screw (23), transforms by virtue of the changing of the direction of its horizontal movement to vertical movement, through the movement slot (30) of the line (34) and, thus, slowly exerts great vertical traction force on the active tips (14), and these on the tooth to be extracted (D1), dislodging it from its alveolus, without traumas.

In the case of extraction of a root, the latter is penetrated by a perpetual screw in whose head one of the ends of the line (35) is secured, while the other end of the line (34) is fixed in any way to the tensioning carriage (21), this operation of root extraction being identical to the extraction of a tooth.

## Claims

1. "ERGONOMIC TOOL FOR ATRAUMATIC TOOTH EXTRACTION", which reduces the duration of tooth extraction procedures from about 40 to 4 minutes and cicatrization to about 3 days, eliminates the countless iatrogenic effects inherent to traditional tooth extraction, protects the alveolar edges from injury, does not cause bleeding, eliminates the possibility of causing lockjaw in the patient, it does not require physical effort of the dental surgeon, has near-zero risk of iatrogenic effects, such as crown breakage and the consequences thereof, as well as temporo-mandibular accidents, it allows delicate interventions that are practically impossible using a forceps, making it possible for teeth to be extracted with the cementoblast intact and treated outside the body with absolute safety, and re-implanted, with very high rates of success, it makes it possible to lift the teeth a few millimeters, with absolute safety, it does not require the use of electric or pneumatic drills for drilling crowns, shortening surgery time and reducing patient discomfort, it has a low cost, since a single tool carries out all tooth extraction or prosthesis and pin withdrawal operations, unlike the present tooling, which requires a collection of more than ten tools, due to the system of universal adjustable pliers with active tips, it allows tooth extraction without support on any of the proximal teeth, it has a small size, allowing the extraction of the third and fourth molars, which uses a system based on applications of the principles of basic machines - levers, screws and lines, without the need for pulleys, **characterized in that** it basically comprises of a set of two parts that are independent and can be coupled together simply by juxtapositioning, of reduced dimensions and having a group of devices, easily adjustable and removable from them, consisting of torque keys, active tips, support bases, a line and its fixing clip; that the holding pliers (1A), has a left shaft (2) and a right shaft (3), active tips (14) which are movable yet held fixed in position, springs (13) that apply pressure to the active tips (14) when inserted in the openings (14A) of the two shafts, and that the necks (15) of the active tips (14) are secured to the tips of the line (34), and also the spring (6) which is inserted in the seats (7), having also a distal end (3A) of the right shaft (3), which has a millimeter screw opening (9), a millimeter nut (8) through which passes, when turned, the millimeter screw (10), joined to the torque device (12), with its slot (13R), for passage of the torque key (13A); the spring (16) and the fast adjustment end (11), joined to the torque device (12).

2. "ERGONOMIC TOOL FOR ATRAUMATIC TOOTH EXTRACTION" according to claim 1, **characterized in that** the torque device (12) is turned until the correct force is applied to the teeth via the active tips (14), which force is a consequence of the effect of the set of two levers, of intermediate support, formed by the left shaft (2) and the right shaft (3), which turn about the support tip realized by the axis of turning (5).

3. "ERGONOMIC TOOL FOR ATRAUMATIC TOOTH EXTRACTION" according to claim 1, **characterized in that** the torque key (13A) passes through the slot (13) of the torque device (12), inside which it is held under pressure by the locking spring (16), so that as the torque device (12) is turned it can easily change position, forward or backward

4. "ERGONOMIC TOOL FOR ATRAUMATIC TOOTH EXTRACTION" according to claim 1, **characterized in that**, when a force is applied to the torque key (13A), the millimeter screw (10) is turned and begins to apply force to the distal end (2A) of the left shaft (2), and this force can be regulated by the experience of the operator or controlled by torque meter (17), placed at the lower end (3A) of the right shaft (3).

5. "ERGONOMIC TOOL FOR ATRAUMATIC TOOTH EXTRACTION" according to claim 1, **characterized in that** the extractor (1 B) has a shaft (18), which widens in its terminal portion, which is outside the patient's mouth and forms the operating handle (19), hollowed out by the window (20), inside which travels the tensioning carriage (21) which, at its upper portion, has the fixator shoulder (22) for the line (34), said fixator shoulder (22) having, in its central portion, a groove (24); and also a millimeter endless screw (23), which passes through the threaded opening (25) of the tensioning carriage (21), a torque device (27), a slot (28) for passage and fixation of the torque key (13A) of the torque device (27), and the fast end (26); a slot (30) for movement of said line (34), three threaded openings (29) where the screws for locking the support bases will pass, and the three springs (29A) that will hold these screws in position, applying pressure to the screws inside the threaded holes (29).

6. "ERGONOMIC TOOL FOR ATRAUMATIC TOOTH EXTRACTION" according to claim 1, **characterized in that** the functioning of the regulating elements of the extractor (1 B) is as follows: the heights at which the latter will be braced against the teeth or gums neighboring the tooth being treated will be determined by two support bases which will be correctly positioned by screws held in place by the springs (29A) in regard to the height and other horizontal adjustments of the surgical field; the tensioning carriage (21) moves in the direction toward the distal part of the extractor (1 B), in the beginning by the rotation of the fast end (26), which is done gently and without the need to exert force, by the operator with the tips of his thumb and index finger, such that the tensioning carriage (21), through which passes the millimeter endless screw (23) that crosses the threaded opening (25) of the tensioning carriage (21), upon turning, causes the latter to move.

7. "ERGONOMIC TOOL FOR ATRAUMATIC TOOTH EXTRACTION" according to claim 1, **characterized in that** the active tips (14) have grooves (14B), the necks (15) and the locating pin (14C), which engage in the openings (14A) of both the left shaft (2) and the right shaft (3), where they are firmly positioned by the action of the springs (13), and also the shoulders (14D) of the locating pins (14C) of the active tips (14), whose functions are to hold the line (34) in correct position between the necks (15) and the shoulders (14D).

8. "ERGONOMIC TOOL FOR ATRAUMATIC TOOTH EXTRACTION" according to claim 1, **characterized in that** the torque key (13A) can operate in the slot (13) or in the movement slot (30) of the line (34), and **in that** the function of said torque key (13A) is a lever to impose torque on the torque device (12) and on the torque device (27), and also **in that** inside these two devices there are, respectively, the spring (16) and the spring (29A), which keep these parts fixed in the positions in which the operators place them, and **in that** these springs permit the movement of the keys, inside the slot (13) or in the slot for movement (30) of the line (34).

9. "ERGONOMIC TOOL FOR ATRAUMATIC TOOTH EXTRACTION" according to claim 1, **characterized in that** the millimeter screw (10) is joined to the torque device (12), with its slot (13) for the passage of the torque key (13A), and the fast adjustment end (11) is joined to the torque device (12) and the spring (16), that the torque key (13A) can assume any position inside the slot (13) and even be retracted.

10. "ERGONOMIC TOOL FOR ATRAUMATIC TOOTH EXTRACTION" according to claim 1, **characterized in that** the shaft (18) of the extractor (1 B) has support bases (31), springs (29A) that serve to hold the screws (32) firmly in the positions determined by the operator, both as regards the heights and the horizontal positions of same; the movement slot (30), through which the line (34) will pass, coming from the tensioning carriage (21), to wrap around the active tips (14) of the holding pliers (1A), screws (32) and threaded openings (29) by which the screws (32) pass through the shaft (18), the support bases (31) with their flexible pads (33), and also a holding clip (35) for the line (34).

11. "ERGONOMIC TOOL FOR ATRAUMATIC TOOTH EXTRACTION" according to the previous claims, **characterized in that** the holding pliers (1 A) and the extractor (1 B) are coupled together by mere juxtapositioning and its operation occurs **in that** the two tips are lashed to the necks (15) of the active tips (14), the line (34) being a device that has its loop
